# EUROPEAN PATENT SPECIFICATION

(11) **EP 3 342 446 B1**
(45) Date of publication and mention of the grant of the patent: **18.01.2023**
(21) Application number: 16839251.2
(22) Date of filing: 23.08.2016
(51) Int. Cl.: A61M 25/10, B29C 49/48, B29K 77/00, B29L 31/00

(54) **BALLOON FOR CATHETER, BALLOON CATHETER, AND MOLDING DIE**
BALLON FÜR EINEN KATHETER, BALLONKATHETER UND GUSSFORM
BALLONNET POUR CATHÉTER, CATHÉTER À BALLONNET, ET MATRICE DE MOULAGE

(30) Priority: 27.08.2015 JP 2015167584
(43) Date of publication of application: 04.07.2018
(73) Proprietor: Goodman Co., Ltd., Aichi 460-0008 (JP)
(72) Inventor: NAKAGAWA, Yuki, Seto-shi Aichi 489-0976 (JP); MIYAMURA, Takuma, Seto-shi Aichi 489-0976 (JP); KAMEOKA, Koji, Seto-shi Aichi 489-0976 (JP); HARA, Jiro, Seto-shi Aichi 489-0976 (JP); MURANO, Seiichiro, Nagoya-shi Aichi 460-0008 (JP)
(74) Representative: Epping - Hermann - Fischer
(86) International application number: PCT/JP2016/074429
(87) International publication number: WO 2017/033904

(56) References cited:
- EP-A1- 1 554 006
- WO-A1-94/28963
- JP-A- S6 399 879
- JP-A- 2000 509 304
- US-A1- 2001 023 335
- US-A1- 2013 060 234

## Description

### Technical Field

The present invention relates to a balloon for a catheter that is joined to a distal end of a catheter shaft, a balloon catheter formed by the balloon for the catheter joined to the distal end of the catheter shaft, and a molding die for forming the balloon for the catheter.

### Background art

A balloon catheter is known that is used in treatments that dilate a constricted location of a blood vessel. Patent Literature 1 discloses a biological organ dilator that is provided with a shaft main body and a balloon. The balloon can be folded over and inflated in accordance with changes in an inner pressure. The balloon is provided on a distal end of the shaft main body. The balloon includes an inflatable part, a distal end-side transition part, a distal end-side joint part, a base end-side transition part, and a base end-side joint part. The inflatable part inflates to a tube shape having substantially the same diameter as a result of fluid being introduced into the balloon. The distal end-side joint part is joined to an inner tube of the shaft main body. The distal end-side transition part is provided between the distal end-side joint part and the inflatable part. The base end-side joint part is joined to an outer tube of the shaft main body. The base end-side transition part is provided between the base end-side joint part and the inflatable part. Each of the distal end-side transition part and the base end-side transition part is provided with a high inclination part on the side of the inflatable part, and with a low inclination part on the side opposite to the side of the inflatable part. The inclination angle of the high inclination part with respect to a lengthwise direction of the balloon is larger than the inclination angle of the low inclination part with respect to the lengthwise direction of the balloon. Hereinafter, the inflatable part is referred to as an "inflation part." The distal end-side transition part and the base end-side transition part are referred to as "connecting parts." The distal end-side joint part and the base end-side joint part are referred to as "joint parts."

The disclosure of EP 1 554 006 A1 relates to a catheter balloon with a cone design. The catheter balloon has at least one cone section with a volume in mm³ such that, when the ratio of one cone volume to the transverse cross-sectional area in mm² of the inflated median section is at least about 2.1 mm. In certain applications, the cone design can assist in the preferential expansion of the cone sections prior to any significant expansion of the median section. The balloon can thus be utilized as a component of a balloon catheter or prosthesis delivery system.

Document US 2013/060234 A1 discloses a catheter including an outer conduit and an inner conduit movably disposed therein. The inner conduit includes at least one movable part. The proximal end of the inner conduit is angled piercing the wall of the outer conduit. The catheter includes an inflatable intussusceptable balloon having a proximal margin attached to the distal tip of the outer conduit and a distal margin attached to a portion of the inner conduit extending beyond the distal tip of the outer conduit. The diameter of a first portion of the balloon is larger than the diameter of other portions thereof.

Document US 2001/023335 A1 discloses a balloon on a distal portion of a balloon angioplasty catheter. The section of the balloon onto which a stent can be mounted has a central segment that is substantially cylindrical in shape that is centered between two segments each having the shape of a sector of a prolate spheroid. The shape for a balloon for a stent delivery catheter provides a more cylindrical shape for the stent after it is implanted in an artery that has a typical distribution of plaque in an arterial stenosis.

In document WO 94/28963 A1 a double-tapered balloon catheter is described. The balloon catheter including a short central cylindrical section with a tapered section on either or both sides thereof. The tapered sections have a gradual reduction in diameter in comparison to the abrupt angular transition of conventional dilatation balloons.

### Citation List

### Patent Literature

Patent Literature 1: Japanese Patent No. 4955162

### Summary of Invention

Normally, when the balloon is in an inflated state, the inclination angle, with respect to an extension direction of the catheter shaft, of a part of the connecting part that is in the vicinity of the inflation part is preferably large. The reason for this is that the larger the inclination angle, the more easily a user can identify a boundary between the inflation part and the connecting part. Further, when the balloon is in the inflated state, the inclination angle, with respect to the extension direction of the catheter shaft, of a part of the connecting part that is in the vicinity of the joint part is preferably large. The reason for this is that the larger the inclination angle, the more appropriately the balloon can be folded.

Here, in the biological organ dilator disclosed in Patent Literature 1, the inclination angle, with respect to the extension direction of the shaft main body, of the part of the connecting part that is in the vicinity of the joint part is small. Thus, a problem arises that sometimes the balloon cannot be folded appropriately.

An object of the present invention is to provide a balloon for a catheter in which a boundary between an inflation part and a connecting part is easily identified and which has favorable foldability, a balloon catheter formed by the balloon for the catheter joined to a distal end of a catheter shaft, and a molding die for forming the balloon for the catheter.

A balloon for a catheter according to a first aspect of the present invention, as described in claim 1, is configured to inflate and deflate in accordance with changes in internal pressure. The balloon for the catheter is characterized by including: an inflation part extending in a cylindrical shape along an extension direction in an inflated state; a connecting part that is a part extending from at least one end of the inflation part, a diameter of one end of the connecting part being larger than a diameter of another end when the inflation part is in the inflated state, the one end being an end connected to the inflation part and the other end being an end on the opposite side to the one end; and a joint part that extends from the other end of the connecting part; wherein the connecting part includes a first connecting part connected to the inflation part at a first point, a second connecting part connected to the joint part at a second point, and a third connecting part disposed between the first connecting part and the second connecting part, connected to the first connecting part at a third point and connected to the second connecting part at a fourth point, and when the inflation part is in the inflated state, both a first angle and a second angle are larger than a third angle, the first angle being an acute angle between a first line segment and the extension direction, the first line segment being a line segment connecting the first point and the third point, the second angle being an acute angle between a second line segment and the extension direction, the second line segment being a line segment connecting the second point and the fourth point, the third angle being an acute angle between a third line segment and the extension direction, the third line segment being a line segment connecting the third point and the fourth point.

According to the first aspect, when the balloon for the catheter is in the inflated state, of the connecting part, the inclination angle of the first connecting part that is in the vicinity of the inflation part may be made relatively large. Thus, the balloon for the catheter may cause a boundary between the inflation part and the connecting part to be easily identified by a user. Further, when the balloon for the catheter is in the inflated state, of the connecting part, the inclination angle of the second connecting part that is in the vicinity of the joint part may be made relatively large. Thus, the balloon for the catheter may be appropriately deflated.

In the first aspect, a length of the third connecting part is longer than a length of the first connecting part and a length of the second connecting part. Thus, when the balloon for the catheter is in a deflated state, a length of the third connecting part in a direction orthogonal to the extension direction may be gradually changed along the extension direction. Thus, the balloon for the catheter may move smoothly inside a blood vessel when in the deflated state.

In the first aspect, the lengths of the first connecting part and the second connecting part may be substantially the same. In this case, the balloon for the catheter may be easily manufactured.

In the first aspect, when the inflation part is in the inflated state, a diameter of the third connecting part may increase toward the first connecting part. In this case, when the balloon for the catheter is in the deflated state, the length of the third connecting part in the direction orthogonal to the extension direction may be gradually increased from the joint part side toward the inflation part side. Thus, the balloon for the catheter may move smoothly inside the blood vessel when in the deflated state.

A balloon catheter according to a second aspect of the present invention includes the balloon for the catheter according to the first aspect, and a catheter shaft extending between a base end and a distal end, the joint part of the balloon for the catheter being connected to the distal end.

According to the second aspect, when the balloon for the catheter is in the inflated state, in the balloon catheter, of the connecting part, the inclination angle of the first connecting part that is in the vicinity of the inflation part may be made relatively large. Thus, the balloon catheter may cause a boundary between the inflation part and the connecting part of the balloon for the catheter to be easily identified by a user. Further, when the balloon for the catheter is in the inflated state, in the balloon catheter, of the connecting part, the inclination angle of the second connecting part that is in the vicinity of the joint part may be made relatively large. Thus, in the balloon catheter, the balloon for the catheter may be appropriately deflated.

A molding die according to a third aspect of the present invention, as described in claim 5, is configured to form a balloon for a catheter using blow molding. The molding die is characterized by including an inner wall forming a cylindrical space extending in an extension direction, wherein the inner wall includes an inflation part having substantially the same diameter along the extension direction, a connecting part that is a part extending from at least one end of the inflation part, a diameter of one end of the connecting part being larger than a diameter of another end, the one end being an end connected to the inflation part and the other end being an end on the opposite side to the one end, and a joint part that extends from the other end of the connecting part, the connecting part includes a first connecting part connected to the inflation part at a first point, a second connecting part connected to the joint part at a second point, and a third connecting part disposed between the first connecting part and the second connecting part, connected to the first connecting part at a third point and connected to the second connecting part at a fourth point, and both a first angle and a second angle are larger than a third angle, the first angle being an acute angle between a first line segment and the extension direction, the first line segment being a line segment connecting the first point and the third point, the second angle being an acute angle between a second line segment and the extension direction, the second line segment being a line segment connecting the second point and the fourth point, the third angle being an acute angle between a third line segment and the extension direction, the third line segment being a line segment connecting the third point and the fourth point.

According to the third aspect, the balloon for the catheter in which, of the connecting part, the inclination angle of the first connecting part that is in the vicinity of the inflation part is relatively large in the inflated state, may be manufactured by blow molding using the molding die. Thus, the manufactured balloon for the catheter may cause the boundary between the inflation part and the connecting part to be easily identified by the user. Further, the balloon for the catheter in which, of the connecting part, the inclination angle of the second connecting part that is in the vicinity of the joint part is relatively large in the inflated state, may be manufactured by blow molding using the molding die. Thus, the manufactured balloon for the catheter may be appropriately deflated.

### Brief description of the drawings

Fig. 1 is a side view of a balloon catheter 10.
Fig. 2 is a side view of a balloon 3 in a deflated state.
Fig. 3 is a perspective view of the balloon 3 in an inflated state.
Fig. 4 is a side view of the balloon 3 in the inflated state.
Fig. 5 is a cross-sectional view of the balloon 3 in the inflated state.
Fig. 6 is a cross-sectional view showing an expanded view of a base end-side cone part 32 of the balloon 3 in the inflated state.
Fig. 7 is a cross-sectional view showing an expanded view of a distal end-side cone part 34 of the balloon 3 in the inflated state.
Fig. 8 is a diagram showing a manufacturing process (a first process) of the balloon 3 using a molding die 6.
Fig. 9 is a diagram showing a manufacturing process (a second process) of the balloon 3 using the molding die 6.
Fig. 10 is a diagram showing the manufacturing process (the second process) of the balloon 3 using the molding die 6.
Fig. 11 is a diagram showing a manufacturing process (a third process) of the balloon 3 using the molding die 6.
Fig. 12 is a side view of a balloon 7 according to a modified example, in an inflated state.

### Description of Embodiments

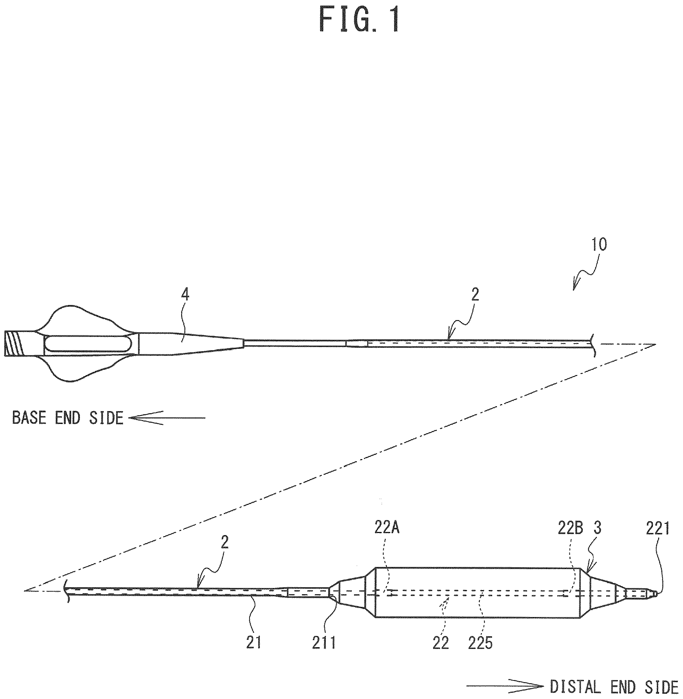

An embodiment of the present invention will be explained with reference to Fig. 1 to Fig. 7. As shown in Fig. 1, a balloon catheter 10 includes a catheter shaft 2, a balloon 3, and a hub 4. The balloon 3 is connected to an end on one side of the catheter shaft 2. The hub 4 is connected to an end on the other side of the catheter shaft 2. Hereinafter, the end (of both ends) on the one side of the catheter shaft 2 is referred to as a "distal end." The end (of both ends) on the other side of the catheter shaft 2 is referred as a "base end." A direction that extends along the catheter shaft 2 is referred to as an "extension direction." Of a direction orthogonal to the extension direction, a side that is in the vicinity of a cross-sectional center of the catheter shaft 2 is referred to as an "inner side" and a side that is separated from the cross-sectional center of the catheter shaft 2 is referred to as an "outer side." A plane that is orthogonal to the extension direction is referred to as a "first plane." A plane that is parallel to the extension direction is referred to as a "second plane."

### Catheter shaft 2, hub 4

As shown in Fig. 1, the catheter shaft 2 has an outer tube 21 and an inner tube 22. The outer tube 21 and the inner tube 22 each have a flexible tubular shape. When cut along the first plane, the diameter of the cross-section the outer tube 21 is larger than the diameter of the cross-section of the inner tube 22 when cut along the first plane. Apart from a predetermined section on the distal end side, the inner tube 22 is disposed inside the outer tube 21. The predetermined section on the distal end side of the inner tube 22 protrudes toward the distal end side from a distal end 211 of the outer tube 21. Thus, a distal end 221 of the inner tube 22 is disposed further to the distal end side than the distal end 211 of the outer tube 21. Hereinafter, the predetermined section on the distal end side of the inner tube 22 is referred to as a "protruding section 225." A guide wire (not shown in the drawings) is inserted into the inner tube 22.

Radiopaque markers (hereinafter referred to simply as "markers) 22A and 22B are mounted on the protruding section 225 of the inner tube 22. A resin into which a radiopaque material is mixed is used as the material of the markers 22A and 22B, for example. The markers 22A and 22B do not allow the passage of radiation. The marker 22B is disposed further to the distal end side than the marker 22A. The markers 22A and 22B are separated from each other in the extension direction.

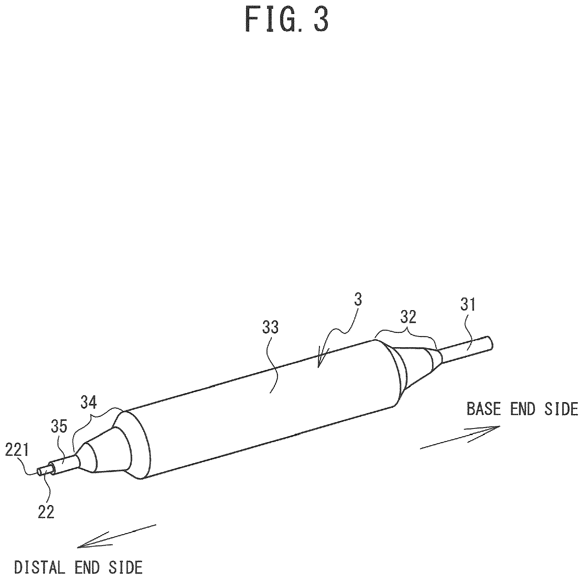

The hub 4 supplies a compressed fluid to the balloon 3, via the outer tube 21. The balloon 3 inflates in accordance with the supply of the compressed fluid (refer to Fig. 3 to Fig. 7).

### Balloon 3

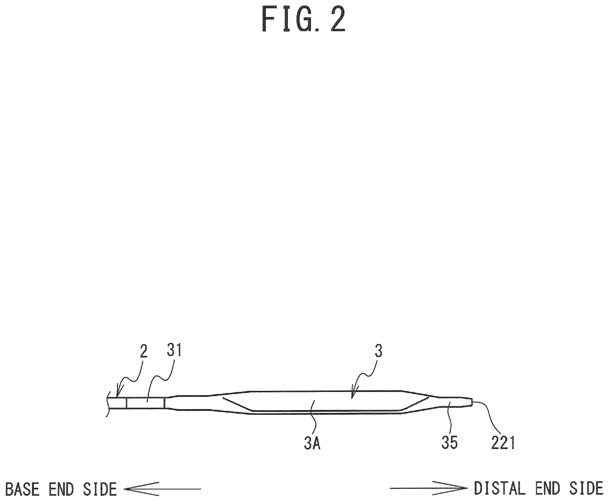

The balloon 3 is configured to inflate and deflate in accordance with changes in internal pressure. As shown in Fig. 2, the balloon 3 deflates to the inner side in a state in which the compressed fluid is not supplied. The balloon 3 is a three pleat type balloon in which three pleats 3A are formed by the balloon 3 in the deflated state. Each of the three pleats 3A is wound around the protruding section 225 (refer to Fig. 1) of the inner tube 22. The pleats 3A are also called "flaps" or "wings." Meanwhile, as shown in Fig. 3 to Fig. 5, the balloon 3 inflates to the outer side when the compressed fluid is supplied. Hereinafter, the balloon 3 in the inflated state will be explained. As shown in Fig. 3, the balloon 3 includes a base end-side leg part 31, a base end-side cone part 32, an inflation part 33, a distal end-side cone part 34, and a distal end-side leg part 35. The base end-side leg part 31, the base end-side cone part 32, the inflation part 33, the distal end-side cone part 34, and the distal end-side leg part 35 respectively correspond to parts of the balloon 3 divided into five parts in the extension direction. The base end-side leg part 31, the base end-side cone part 32, the inflation part 33, the distal end-side cone part 34, and the distal end-side leg part 35 are aligned in that order from the base end side to the distal end side.

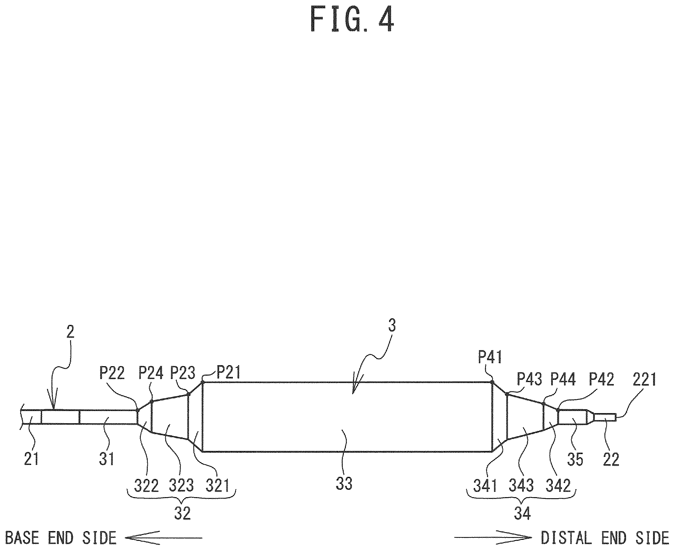

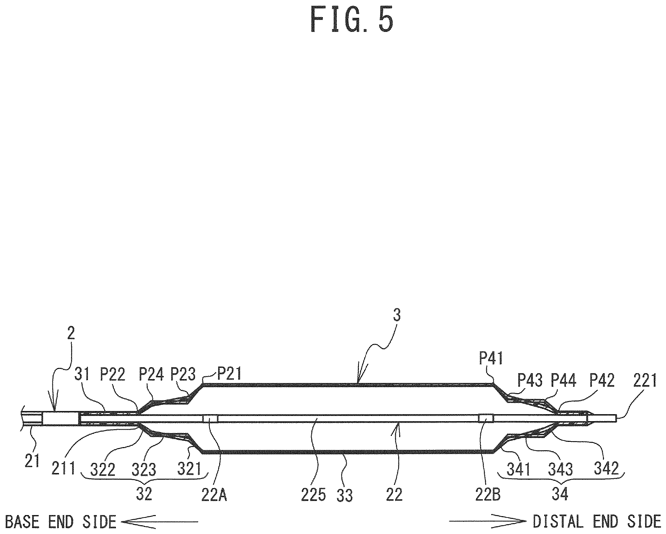

The base end-side leg part 31 extends from the end on the base end side of the base end-side cone part 32. As shown in Fig. 4 and Fig. 5, the base end-side leg part 31 is connected to a section of the outer tube 21 that is further to the base end side than the distal end 211 (refer to Fig. 5). The base end-side leg part 31 is connected to the outer tube 21 by thermal welding, for example. A shape of the base end-side leg part 31 is a circular cylindrical shape that extends in the extension direction. When cut along the first plane, the diameter of the cross-section of the base end-side leg part 31 is substantially the same over the entire length in the extension direction. The base end-side cone part 32 is adjacent to the distal end side of the base end-side leg part 31. The base end-side cone part 32 includes three connecting parts (a first connecting part 321, a second connecting part 322, and a third connecting part 323, to be described later). When cut along the first plane, the diameter of the cross-section of the base end-side cone part 32 becomes larger from the base end side toward the distal end side. The base end-side cone part 32 will be described in detail later. The inflation part 33 is adjacent to the distal end side of the base end-side cone part 32. The shape of the inflation part 33 is a circular cylindrical shape that extends in the extension direction. When cut along the first plane, the diameter of the cross-section the inflation part 33 is substantially the same over the entire length in the extension direction. The outer diameter of the inflation part 33 is a maximum diameter of the balloon 3.

The distal end-side cone part 34 is adjacent to the distal end side of the inflation part 33. The distal end-side cone part 34 includes three connecting parts (a first connecting part 341, a second connecting part 342, and a third connecting part 343, to be described later). When cut along the first plane, the diameter of the cross-section of the distal end-side cone part 34 becomes smaller from the base end side toward the distal end side. The distal end-side cone part 34 will be described in detail later. The distal end-side leg part 35 is adjacent to the distal end side of the distal end-side cone part 34. The distal end-side leg part 35 extends from the distal end of the distal end-side cone part 34. The distal end-side leg part 35 is connected to a section of the protruding section 225 (refer to Fig. 5) of the inner tube 22 that is further to the base end side than the distal end 221. The distal end-side leg part 35 is connected to the protruding section 225 of the inner tube 22 by thermal welding, for example. When cut along the first plane, the diameter of the cross-section of the distal end-side leg part 35 is substantially the same over the entire length in the extension direction. The base end-side cone part 32, the inflation part 33, the distal end-side cone part 34, and the distal end-side leg part 35 cover the protruding section 225 of the inner tube 22 from the outside.

The length of the inflation part 33 in the extension direction is longer than the respective lengths in the extension direction of the base end-side leg part 31, the base end-side cone part 32, the distal end-side cone part 34, and the distal end-side leg part 35. As shown in Fig. 5, the position of the end on the base end side of the inflation part 33 is aligned, in the extension direction, with the position of the end on the base end side of the marker 22A. The position of the end on the distal end side of the inflation part 33 is aligned, in the extension direction, with the position of the end on the distal end side of the marker 22B.

When cut along the first plane, the diameters of the cross-sections of the base end-side leg part 31 and the distal end-side leg part 35 are substantially the same. When cut along the first plane, the diameters of the cross-sections of the base end-side cone part 32 and the distal end-side cone part 34 are different for each of the three connecting parts, but in all cases, the diameters thereof are larger than the diameters of the base end-side leg part 31 and the distal end-side leg part 35 when cut along the first plane. When cut along the first plane, the diameter of the cross-section of the inflation part 33 is larger than the diameters of the base end-side cone part 32 and the distal end-side cone part 34 when cut along the first plane.

When the balloon 3 in the inflated state is cut along the first plane, the thickness of each of sections of the balloon 3 becomes thinner the larger the diameter of the cross-section thereof. Thus, the thicknesses of the base end-side leg part 31 and the distal end-side leg part 35 are thicker than the thicknesses of the other sections of the balloon 3. The thickness of the inflation part 33 is thinner than the thicknesses of the other sections of the balloon 3. The thicknesses of the base end-side cone part 32 and the distal end-side cone part 34 are different for each of the three connecting parts, but in all cases, the thicknesses are thinner than the thicknesses of the base end-side leg part 31 and the distal end-side leg part 35 and are thicker than the thickness of the inflation part 33.

The material of the balloon 3 is a polyamide resin. Note that the material of the balloon 3 is not limited to the polyamide resin, and can be changed to another flexible material. For example, a polyethylene resin, a polypropylene resin, a polyurethane resin, a polyimide resin, silicone rubber, natural rubber or the like may be used as the material of the balloon 3.

### Base end-side cone part 32

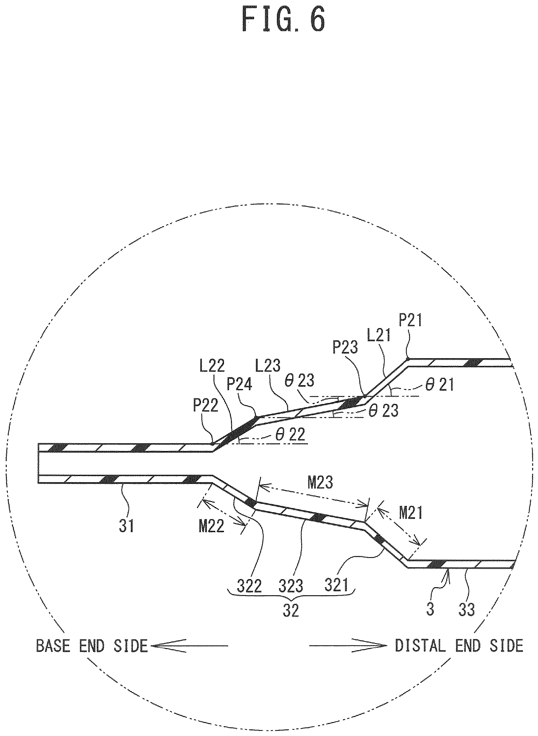

As shown in Fig. 6, the shape of each of the first connecting part 321, the second connecting part 322, and the third connecting part 323 of the base end-side cone part 32 is a truncated cone shape that extends in the extension direction and that has a diameter that widens toward the distal end side when cut along the first plane. The first connecting part 321 is connected to the end on the base end side of the inflation part 33. The first connecting part 321 extends toward the base end side from the connecting section with the inflation part 33. The second connecting part 322 is connected to the end on the distal end side of the base end-side leg part 31. The second connecting part 322 extends toward the distal end side from the connecting section with the base end-side leg part 31. The third connecting part 323 is connected to the end on the base end side of the first connecting part 321 and to the end on the distal end side of the second connecting part 322. The third connecting part 323 extends between the respective connecting sections of the third connecting part 323 with the first connecting part 321 and the second connecting part 322.

When cut along the first plane, the diameter of the cross-section of the end on the distal end side of the first connecting part 321 is larger than the diameter of the cross-section of the end on the base end side of the first connecting part 321 when cut along the first plane. When cut along the first plane, the diameter of the cross-section of the end on the distal end side of the third connecting part 323 is larger than the diameter of the cross-section of the end on the base end side of the third connecting part 323 when cut along the first plane. When cut along the first plane, the diameter of the cross-section of the end on the distal end side of the second connecting part 322 is larger than the diameter of the cross-section of the end on the base end side of the second connecting part 322 when cut along the first plane. When cut along the first plane, the diameters of the cross-sections of the first connecting part 321, the second connecting part 322, and the third connecting part 323 all increase from the base end side toward the distal end side. Thus, when cut along the first plane, the diameter of the cross-section of the end on the distal end side of the base end-side cone part 32 is larger than the diameter of the cross-section of the end on the base end side of the base end-side cone part 32 when cut along the first plane.

When cut along the first plane, the thickness of the base end-side cone part 32 becomes thinner the larger the diameter of the cross-section thereof, and thus, becomes thinner from the base end side toward the distal end side. Further, the thickness of the base end-side cone part 32 becomes increasingly thinner in the order of the second connecting part 322, the third connecting part 323 and the first connecting part 321. In addition, the thicknesses of each of the first connecting part 321, the second connecting part 322, and the third connecting part 323 become increasingly thinner from the base end side toward the distal end side.

When the balloon 3 is cut along the second plane, each of the cross-sections of the first connecting part 321, the second connecting part 322, and the third connecting part 323 extend in a straight line in a direction that is inclined with respect to the extension direction. When cut along the second plane, the length of the cross-section of the first connecting part 321 is referred to as a "first connecting length M21." When cut along the second plane, the length of the cross-section of the second connecting part 322 is referred to as a "second connecting length M22." When cut along the second plane, the length of the cross-section of the third connecting part 323 is referred to as a "third connecting length M23." In this case, the first connecting length M21 and the second connecting length M22 are substantially the same. The third connecting length M23 is substantially twice the length of the first connecting length M21 and the second connecting length M22.

When cut along the second plane, in the cross-section of the balloon 3, a point at which the first connecting part 321 and the inflation part 33 are connected is referred to as a "first point P21." A point at which the second connecting part 322 and the base end-side leg part 31 are connected is referred to as a "second point P22." A point at which the first connecting part 321 and the third connecting part 323 are connected is referred to as a "third point P23." A point at which the second connecting part 322 and the third connecting part 323 are connected is referred to as a "fourth point P24." A line segment joining the first point P21 and the third point P23 is referred to as a "first line segment L21." The first line segment L21 extends along the first connecting part 321. A line segment joining the second point P22 and the fourth point P24 is referred to as a "second line segment L22." The second line segment L22 extends along the second connecting part 322. A line segment joining the third point P23 and the fourth point P24 is referred to as a "third line segment L23." The third line segment L23 extends along the third connecting part 323. An acute angle, of angles formed between the first line segment L21 and the extension direction, is referred to as a "first angle θ21." An acute angle, of angles formed between the second line segment L22 and the extension direction, is referred to as a "second angle θ22." An acute angle, of angles formed between the third line segment L23 and the extension direction, is referred to as a "third angle θ23." Specific values of the first angle θ21, the second angle 022, and the third angle θ23 are, respectively, 45 degrees, 30 degrees, and 10 degrees. The first angle θ21 and the second angle θ22 are both larger the third angle θ23. Further, the first angle θ21 is larger than the second angle θ22.

### Distal end-side cone part 34

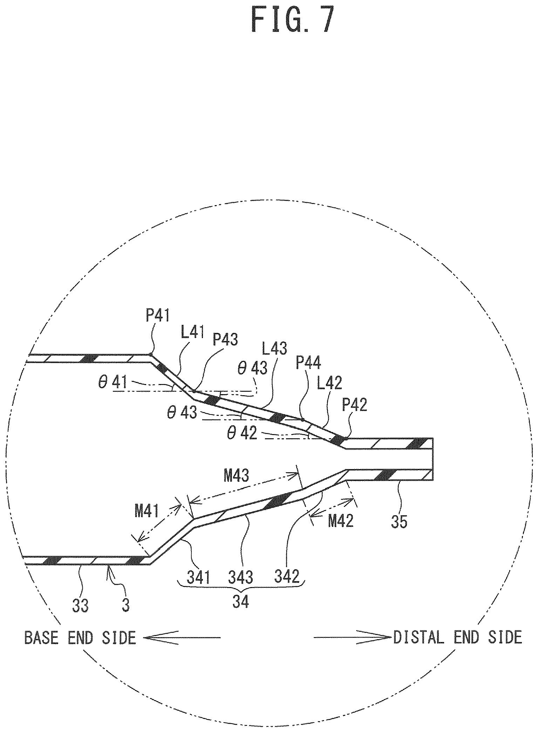

As shown in Fig. 7, the distal end-side cone part 34 is symmetrical to the base end-side cone part 32 (refer to Fig. 6) in the extension direction. The first connecting part 341, the second connecting part 342, and the third connecting part 343 of the distal end-side cone part 34 correspond, respectively, to the first connecting part 321, the second connecting part 322, and the third connecting part 323 (refer to Fig. 6) of the base end-side cone part 32. The first connecting part 341 is connected to the end on the distal end side of the inflation part 33. The first connecting part 341 extends toward the distal end side from the connecting section with the inflation part 33. The second connecting part 342 is connected to the end on the base end side of the distal end-side leg part 35. The second connecting part 342 extends toward the base end side from the connecting section with the distal end-side leg part 35. The third connecting part 343 is connected to the end on the distal end side of the first connecting part 341 and the end on the base end side of the second connecting part 342. The third connecting part 343 extends between the respective connecting sections of the third connecting part 343 with the first connecting part 341 and the second connecting part 342. When cut along the first plane, the diameters of the cross-sections of the first connecting part 341, the second connecting part 342, and the third connecting part 343 all increase from the distal end side toward the base end side. Thus, when cut along the first plane, the diameter of the cross-section of the end on the base end side of the distal end-side cone part 34 is larger than the diameter of the cross-section of the end on the distal end side of the distal end-side cone part 34 when cut along the first plane.

A first connecting length M41, a second connecting length M42, and a third connecting length M43 of the distal end-side cone part 34 respectively correspond to the first connecting length M21, the second connecting length M22, and the third connecting length M23 (refer to Fig. 6) of the base end-side cone part 32. The first connecting length M41 and the second connecting length M42 are substantially the same. The third connecting length M43 is substantially twice the length of the first connecting length M41 and the second connecting length M42.

A first point P41, a second point P42, a third point P43, and a fourth point P44 of the distal end-side cone part 34 respectively correspond to the first point P21, the second point P22, the third point P23, and the fourth point P24 (refer to Fig. 6) of the base end-side cone part 32. A first line segment L41, a second line segment L42, and a third line segment L43 of the distal end-side cone part 34 respectively correspond to the first line segment L21, the second line segment L22, and the third line segment L23 of the base end-side cone part 32. A first angle θ41, a second angle θ42, and a third angle θ43 of the distal end-side cone part 34 respectively correspond to the first angle θ21, the second angle θ22, and the third angle θ23 of the base end-side cone part 32. The first angle θ41 and the second angle θ42 are both larger than the third angle θ43. Further, the first angle θ41 is larger than the second angle θ42.

### Manufacturing method of balloon 3

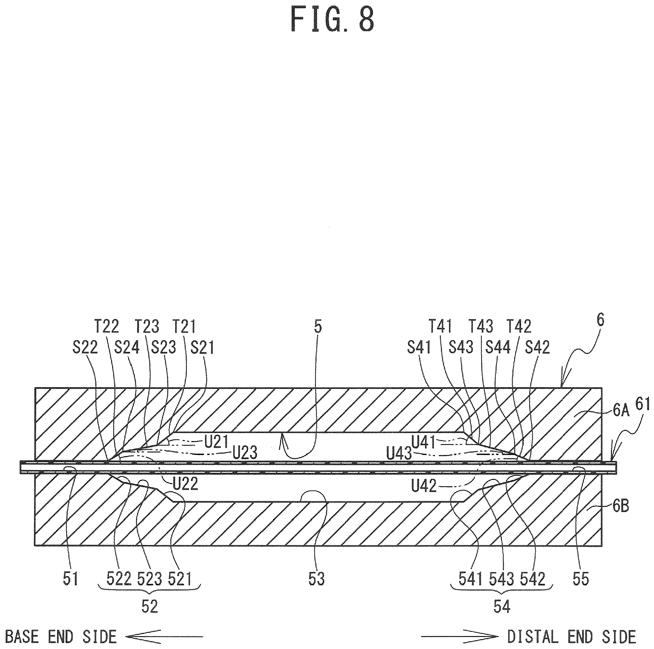

A manufacturing method of the balloon 3 will be explained with reference to Fig. 8 to Fig. 11. The balloon 3 is manufactured by blow molding using a molding die 6. Hereinafter, the left-right direction of the molding die 6 in Fig. 8 to Fig. 11 is referred to as the "extension direction." The left side of the molding die 6 in Fig. 8 to Fig. 11 is referred to as the "base end side," and the right side is referred to as the "distal end side." In the same way as the case of the balloon 3, the plane orthogonal to the extension direction is referred to as the "first plane." In the same way as the case of the balloon 3, the direction parallel to the extension direction is referred to as the "second plane."

The molding die 6 includes a pair of mating dies 6A and 6B. In a state in which the pair of mating dies 6A and 6B are fitted together, the molding die 6 includes inner walls 5. The inner walls 5 form a substantially circular cylindrical space that extends in the extension direction inside the molding die 6. When the molding die 6 is cut along the second plane, the shape of the cross-section of the inner walls 5 substantially matches the shape of the cross-section (refer to Fig. 5) of the balloon 3 in the inflated state when cut along the second plane. The inner walls 5 include a base end-side leg part 51, a base end-side cone part 52, an inflation part 53, a distal end-side cone part 54 and a distal end-side leg part 55. The base end-side cone part 52 includes a first connecting part 521, a second connecting part 522, and a third connecting part 523. The distal end-side cone part 54 includes a first connecting part 541, a second connecting part 542, and a third connecting part 543. The base end-side leg part 51, the base end-side cone part 52, the inflation part 53, the distal end-side cone part 54, and the distal end-side leg part 55 of the inner walls 5 of the molding die 6 correspond to the base end-side leg part 31, the base end-side cone part 32, the inflation part 33, the distal end-side cone part 34, and the distal end-side leg part 35 (refer to Fig. 5) of the balloon 3. The first connecting part 521, the second connecting part 522, and the third connecting part 523 of the inner walls 5 of the molding die 6 respectively correspond to the first connecting part 321, the second connecting part 322, and the third connecting part 323 of the balloon 3. The first connecting part 541, the second connecting part 542, and the third connecting part 543 of the inner walls 5 of the molding die 6 respectively correspond to the first connecting part 341, the second connecting part 342, and the third connecting part 343 of the balloon 3.

When cut along the first plane, the diameters of the cross-sections of the first connecting part 521, the second connecting part 522, and the third connecting part 523 of the base end-side cone part 52 all increase from the base end side toward the distal end side. Thus, when cut along the first plane, the diameter of the cross-section of the end on the distal end side of the base end-side cone part 52 is larger than the diameter of the cross-section of the end on the base end side of the base end-side cone part 52 when cut along the first plane. When cut along the first plane, the diameters of the cross-sections of the first connecting part 541, the second connecting part 542, and the third connecting part 543 of the distal end-side cone part 54 all increase from the distal end side toward the base end side. Thus, when cut along the first plane, the diameter of the cross-section of the end on the base end side of the distal end-side cone part 54 is larger than the diameter of the cross-section of the end on the distal end side of the distal end-side cone part 54 when cut along the first plane.

As shown in Fig. 8, a first point S21, a second point S22, a third point S23, and a fourth point S24 of the base end-side cone part 52 of the inner walls 5 respectively correspond to the first point P21, the second point P22, the third point P23, and the fourth point P24 (refer to Fig. 6) of the base end-side cone part 32 of the balloon 3. A first line segment T21, a second line segment T22, and a third line segment T23 shown in Fig. 8 respectively correspond to the first line segment L21, the second line segment L22, and the third line segment L23 shown in Fig. 6. A first angle U21, a second angle U22 and a third angle U23 shown in Fig. 8 respectively correspond to the first angle θ21, the second angle θ22, and the third angle θ23 shown in Fig. 6. The first angle U21 and the second angle U22 are both larger than the third angle U23. Further, the first angle U21 is larger than the second angle U22.

A first point S41, a second point S42, a third point S43, and a fourth point S44 of the distal end-side cone part 54 of the inner walls 5 respectively correspond to the first point P41, the second point P42, the third point P43, and the fourth point P44 (refer to Fig. 7) of the distal end-side cone part 34 of the balloon 3. A first line segment T41, a second line segment T42, and a third line segment T43 shown in Fig. 8 respectively correspond to the first line segment L41, the second line segment L42, and the third line segment L43 shown in Fig. 7. A first angle U41, a second angle U42, and a third angle U43 shown in Fig. 8 respectively correspond to the first angle θ41, the second angle θ42, and the third angle θ43 shown in Fig. 7. The first angle U41 and the second angle U42 are both larger than the third angle U43. Further, the first angle U41 is larger than the second angle U42.

The blow molding using the molding die 6 is performed through the following first process to third process. The first process will be explained. The pair of mating dies 6A and 6B of the molding die 6 are disposed in a state of being separated from each other. A cylindrical parison 61 formed of a plasticized elastic material is extruded between the mating dies 6A and 6B from an extruding machine (not shown in the drawings). Next, as shown in Fig. 8, the pair of mating dies 6A and 6B disposed so as to be separated from each other are fitted together. The parison 61 is clamped by the pair of mating dies 6A and 6B. The end on the distal end side of the parison 61 is closed.

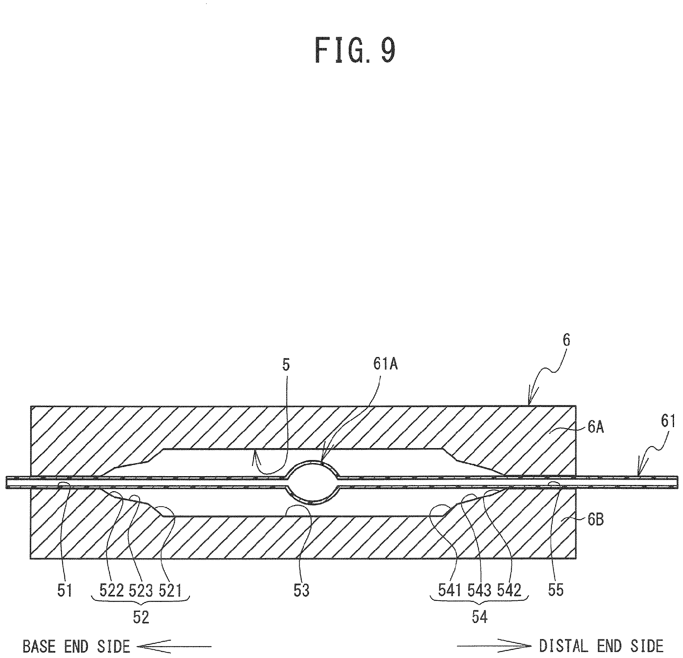

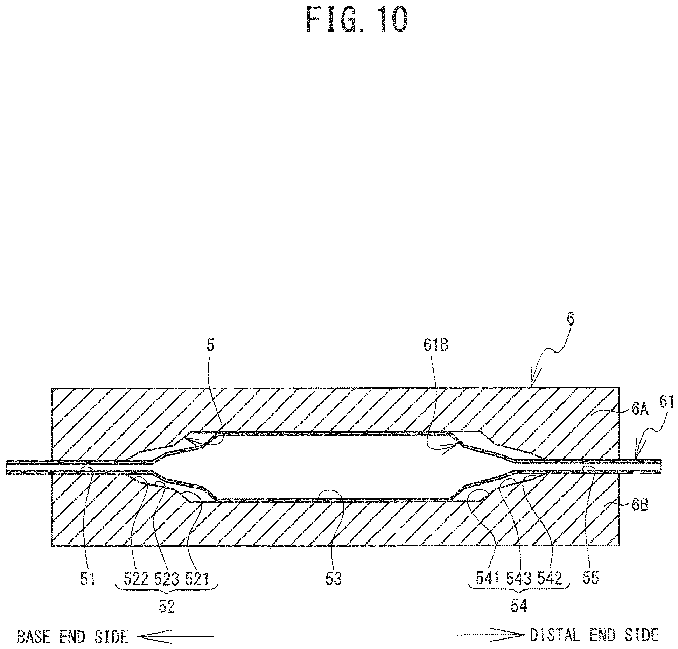

The second process will be explained. An air blowing needle (not shown in the drawings) is inserted into the parison 61 from the end on the base end side of the parison 61. Air is blown into the parison 61 from the air blowing needle. As shown in Fig. 9, as a result of the blown in air, the parison 61 starts to expand from a central section 61A in the extension direction. As shown in Fig. 10, in accordance with an increase in the blown in air, an expanded section 61B becomes larger, and is pressed against the inner walls 5 of the molding die 6. Finally, all of the sections of the parison 61 disposed inside the molding die 6 are pressed against the inner walls 5.

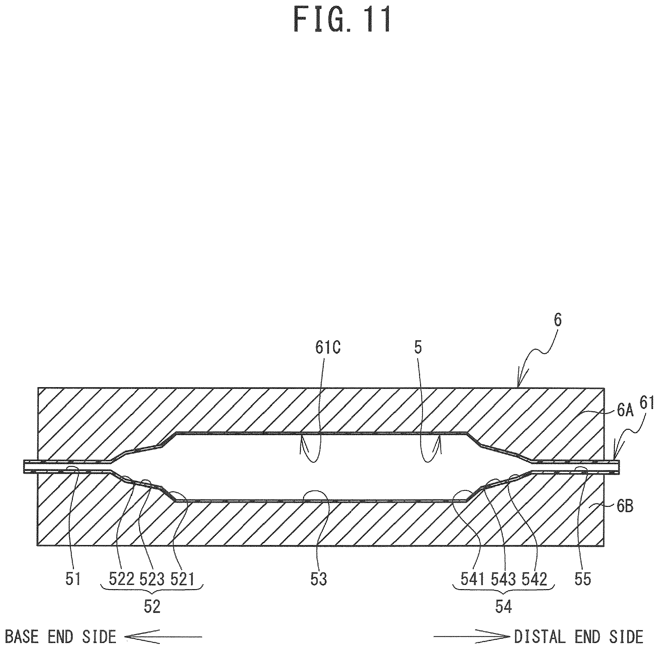

The third process will be explained. As shown in Fig. 11, when a state is obtained in which an expanded section 61C, which is all of the section of the parison 61 that is disposed inside the molding die 6, is pressed against the inner walls 5, the filling of the air from the air blowing needle is stopped. The expanded section 61C of the parison 61 is solidified by cooling. After cooling, the air is extracted from the expanded section 61C. The pair of mating dies 6A and 6B of the molding die 6 are separated from each other and the expanded section 61C is removed. Burrs and the like are removed from the expanded section 61C. Note that, when the molding die 6 is cut along the second plane, the shape of the inner walls 5 substantially matches the shape of the cross-section of the balloon 3 in the inflated state (refer to Fig. 5) when cut along the second plane. Thus, the balloon 3 is obtained as a result the blow molding using the molding die 6 (the first process to the third process).

### Main operations and effects of present embodiment

As explained above, of the inflated balloon 3, the angle θ21 between the extension direction and the first line segment L21 that extends along the first connecting part 321 of the base end-side cone part 32 is larger than the third angle θ23 between the extension direction and the third line segment L23 that extends along the third connecting part 323. Thus, of the three connecting parts of the base end-side cone part 32, an inclination angle of the first connecting part 321, which is connected to the inflation part 33, with respect to the inflation part 33 can be made relatively large. Further, the first angle θ41 between the extension direction and the first line segment L41 that extends along the first connecting part 341 of the distal end-side cone part 34 is larger than the third angle θ43 between the extension direction and the third line segment L43 that extends along the third connecting part 343. Thus, of the three connecting parts of the distal end-side cone part 34, an inclination angle of the first connecting part 341, which is connected to the inflation part 33, with respect to the inflation part 33 can be made relatively large. As a result, respective boundaries between the inflation part 33 that extends in the extension direction in the inflated state and the first connecting parts 321 and 341 can easily be identified, in accordance with the inclination angles thereof being made relatively large. Therefore, a user of the balloon 3 can easily identify each of the boundaries between the inflation part 33 and the base end-side cone part 32, and the inflation part 33 and the distal end-side cone part 34.

Normally, the thinner the thickness of the balloon 3, the more easily it bends, and the balloon 3 deflates easily and appropriately in a state in which the compressed fluid is not supplied. Here, the thickness of the balloon 3 becomes thinner, the larger the diameter of the cross-section of the balloon 3 in the inflated state when cut along the first plane. The reason for this is that in the blow molding, the balloon 3 is manufactured by the parison 61 being dilated using air, and thus, the greater the degree of dilation, the more the parison 61 expands and the thinner the thickness becomes. Further, the greater the inclination angle with respect to the extension direction in the balloon 3, the more rapid a change in the diameter of the cross-section thereof. As a result, when cut along the first plane, the more rapid the increase in the cross-section of the balloon 3 in the inflated state, the more rapidly the thickness of the balloon 3 becomes thinner.

Meanwhile, of the balloon 3 in the inflated state, the second angle θ22 between the extension direction and the second line segment L22 that extends along the second connecting part 322 of the base end-side cone part 32 is larger than the third angle θ23 between the extension direction and the third line segment L23 that extends along the third connecting part 343. Thus, of the base end-side cone part 32, by making the inclination angle of the second connecting part 322 that is connected to the base end-side leg part 31 relatively large, the thickness of the balloon 3 can be made thinner at this section. Further, the second angle θ42 between the extension direction and the second line segment L42 that extends along the second connecting part 342 of the distal end-side cone part 34 is larger than the third angle θ43 between the extension direction and the third line segment L43 that extends along the third connecting part 343. Thus, of the three connecting parts of the distal end-side cone part 34, by making the inclination angle of the second connecting part 342 that is connected to the distal end-side leg part 35 relatively large, the thickness of the balloon 3 can be made thinner at this section. As a result, the balloon 3 can be caused to appropriately deflate at these sections.

Meanwhile, the inclination angles with respect to the extension direction of each of the base end-side cone part 32 and the distal end-side cone part 34 are preferably small, from the point of view of the movability of the balloon 3. This is because, the smaller the inclination angle, the more gradually the length, in the direction orthogonal to the extension direction, of each of the base end-side cone part 32 and the distal end-side cone part 34 when the balloon 3 is in the deflated state can be caused to change along the extension direction. In this case, the balloon 3 can be moved smoothly inside a blood vessel. Here, the base end-side cone part 32 of the balloon 3 has the third connecting part 323 and the distal end-side cone part 34 has the third connecting part 343. The third angle θ23 between the extension direction and the third line segment L23 that extends along the third connecting part 323 is smaller than the first angle θ21 and the second angle θ22. Further, the third angle θ43 between the extension direction and the third line segment L43 that extends along the third connecting part 343 is smaller than the first angle θ41 and the second angle θ42. Thus, in the balloon 3, due to these sections, the inclination angles of each of the base end-side cone part 32 and the distal end-side cone part 34 with respect to the extension direction can be made smaller. As a result, in the balloon 3, the boundaries of the inflation part 33 can be easily identified using the first connecting parts 321 and 341, and the deflatability of the balloon 3 can be appropriately realized due to the second connecting parts 322 and 342. At the same time, the balloon 3 can be moved favorably inside the blood vessel due to the third connecting parts 323 and 343.

The third connecting length M23 that is the length of the connecting part 323 of the base end-side cone part 32 is longer than the first connecting length M21 that is the length of the first connecting part 321 and the second connecting length M22 that is the length of the second connecting part 322. The third connecting length M43 that is the length of the third connecting part 343 of the distal end-side cone part 34 is longer than the first connecting length M41 that is the length of the first connecting part 341 and the second connecting length M42 that is the length of the second connecting part 342. In this case, the length of each of the third connecting parts 323 and 343, in the direction orthogonal to the extension direction, when the balloon 3 is in the deflated state changes gradually along the extension direction. Thus, the balloon for the catheter can be moved smoothly inside the blood vessel.

The first connecting length M21 of the first connecting part 321 of the base end-side cone part 32 is substantially the same as the second connecting length M22. Further, the first connecting length M41 of the first connecting part 341 of the distal end-side cone part 34 is substantially the same as the second connecting length M42. In this case, the shape of each of the first connecting part 521 and the second connecting part 522, and of the first connecting part 541 and the second connecting part 542, in the molding die 6, can be caused to be the same shape. In this case, the manufacture of the molding die 6 is easy, and thus the balloon 3 can be easily manufactured.

The diameter of the end on the distal end side of the base end-side cone part 32 is larger than the diameter of the end on the base end side of the base end-side cone part 32. In this case, the length of the base end-side cone part 32, in the direction orthogonal to the extension direction, when the balloon 3 is in the deflated state can be caused to gradually increase from the base end side toward the distal end side. Further, the diameter of the end on the base end side of the distal end-side cone part 34 is larger than the diameter of the end on the distal end side of the distal end-side cone part 34. In this case, the length of the distal end-side cone part 34, in the direction orthogonal to the extension direction, when the balloon 3 is in the deflated state can be caused to gradually increase from the distal end side toward the base end side. In this manner, the balloon 3 is not likely to be caught up inside the blood vessel, in whichever direction the balloon 3 is moving (to the base end side and the distal end side), and thus, the balloon 3 can be caused to move even more smoothly inside the blood vessel.

The balloon 3 is manufactured by the blow molding using the molding die 6. The shape of the cross-section of the inner walls 5 of the molding die 6 is substantially the same as the shape of the cross section of the balloon 3. Thus, the balloon 3 can be accurately and efficiently manufactured by using the molding die 6.

### Modified examples

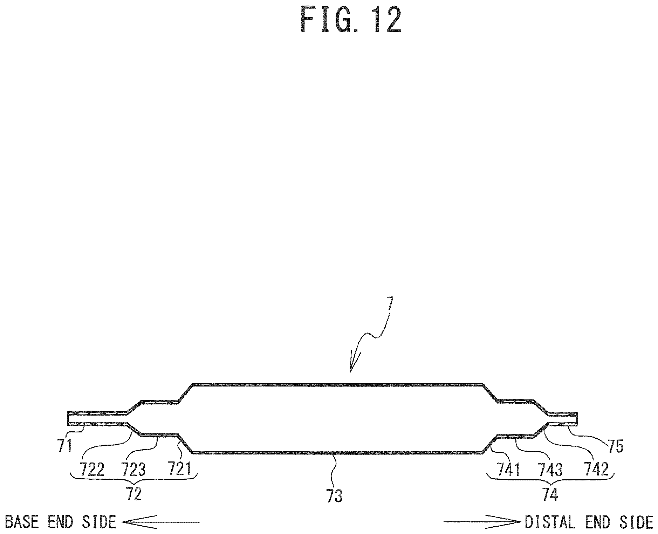

The present invention is not limited to the above-described embodiment and various modifications can be made. A balloon 7 according to a modified example will be explained with reference to Fig. 12. The balloon 7 includes a base end-side leg part 71, a base end-side cone part 72, an inflation part 73, a distal end-side cone part 74, and a distal end-side leg part 75. The base end-side cone part 72 includes three connecting parts (a first connecting part 721, a second connecting part 722, and a third connecting part 723). The distal end-side cone part 74 includes three connecting parts (a first connecting part 741, a second connecting part 742, and a third connecting part 743). The base end-side leg part 71, the base end-side cone part 72, the inflation part 73, the distal end-side cone part 74, and the distal end-side leg part 75 correspond to the base end-side leg part 31, the base end-side cone part 32, the inflation part 33, the distal end-side cone part 34, and the distal end-side leg part 35 of the balloon 3 (refer to Fig. 5). The first connecting parts 721 and 741, the second connecting parts 722 and 742, and the third connecting parts 723 and 743 correspond to the first connecting parts 321 and 341, the second connecting parts 322 and 342, and the third connecting parts 323 and 343 of the balloon 3. The respective lengths of the first connecting parts 721 and 741 are the same as the first connecting lengths M21 and M41. The respective lengths of the second connecting parts 722 and 742 are the same as the second connecting lengths M22 and M42. The respective lengths of the third connecting parts 723 and 743 are the same as the third connecting lengths M23 and M43. A point of difference between the balloon 7 and the balloon 3 is that, when the balloon 7 is cut along the first plane, the cross-sections of the third connecting parts 723 and 743 extend along the extension direction. The remaining configuration of the balloon 7 is the same as that of the balloon 3 and an explanation thereof is omitted here.

In the case of the balloon 7, the lengths of the first connecting parts 721 and 741 in the extension direction and the lengths of the second connecting parts 722 and 742 in the extension direction can be maintained to be approximately the same as in the balloon 3. On the other hand, in the case of the balloon 7, the lengths of the third connecting parts 723 and 743 in the extension direction can be longer than in the balloon 3. The reason for this is that, while the third connecting parts 323 and 343 of the balloon 3 are inclined with respect to the extension direction, the third connecting parts 723 and 743 of the balloon 7 extend along the extension direction. In this case, when the balloon 7 is in a deflated state, the lengths, in the direction orthogonal to the extension direction, of each of the third connecting parts 723 and 743 of the balloon 7 are constant along the extension direction. Thus, in the balloon 7, the user can easily identify the boundaries of the base end-side cone part 72 and the distal end-side cone part 74 with the inflation part 73, using the first connecting parts 721 and 741, and at the same time, the balloon 7 in the deflated state can be moved smoothly inside the blood vessel, due to the third connecting parts 723 and 743.

When cut along the first plane, the diameter of the cross-section of the inflation part 33 of the balloon 3 is substantially the same over the entire length in the extension direction. Thus, when cut along the second plane, the cross-section of the inflation part 33 extends in a straight line along the extension direction. In contrast to this, when cut along the second plane, the cross-section of the inflation part 33 may bend. When cut along the first plane, the diameter of the cross-section of the inflation part 33 may be smaller at both ends in the extension direction, and may be largest at the center in the extension direction.

The base end-side cone part 32 of the balloon 3 includes the first connecting part 321 to the third connecting part 323. Further, the distal end-side cone part 34 includes the first connecting part 341 to the third connecting part 343. In contrast to this, in the balloon 3, it is sufficient that one of the base end-side cone part 32 and the distal end-side cone part 34 includes the first connecting part to the third connecting part, and the other need not necessarily include the first connecting part to the third connecting part.

When cut along the second plane, the cross-section of the first connecting part 321 to the third connecting part 323 of the base end-side cone part 32 is a straight line shape. When cut along the second plane, the cross-section of the first connecting part 341 to the third connecting part 343 of the distal end-side cone part 34 is a straight line shape. In contrast to this, when cut along the second plane, the cross-section of the first connecting part 321 to the third connecting part 323 and of the first connecting part 341 to the third connecting part 343 may be a bent line shape or may be a curved line. In these cases, the first connecting part 321 to the third connecting part 323 need not necessarily be disposed on the first line segment L21 to the third line segment L23. The first connecting part 341 to the third connecting part 343 need not necessarily be disposed on the first line segment L41 to the third line segment L43.

More specifically, for example, when cut along the first plane, the diameters of the cross-sections of the first connecting part 321 to the third connecting part 323 may become increasingly larger from the end on the base end side toward the distal end side, then become increasingly smaller toward the distal end side and then, become increasingly larger toward the distal end side until reaching the end on the distal end side. Further, for example, when cut along the first plane, a rate of change of the diameters of the cross-sections of the first connecting part 321 to the third connecting part 323 may be a curved line that increasingly changes from the base end side toward the distal end side. Note that modified examples of the first connecting part 341 to the third connecting part 343 of the distal end-side cone part 34 are the same as those for the first connecting part 321 to the third connecting part 323 of the base end-side cone part 32 and an explanation thereof is omitted here.

The specific values of the first angle θ21 to the third angle θ23 are examples, and may be other values. The first angle θ21 is larger than the second angle θ22. In contrast to this, the first angle θ21 may be the same as the second angle 022, or the second angle θ22 may be larger than the first angle θ21.

The third connecting length M23 of the third connecting part 323 of the base end-side cone part 32 is longer than the first connecting length M21 of the first connecting part 321 and the second connecting length M22. The first connecting length M21 and the second connecting length M22 are substantially the same. The first connecting length M21 and the second connecting length M22 may be different. The relationships between the first connecting length M41, the second connecting length M42 and the third connecting length M43 of the distal end-side cone part 34 are also the same as the relationships between the first connecting length M21, the second connecting length M22, and the third connecting length M23 and an explanation thereof is omitted here.

Sections of the base end-side cone part 32 corresponding to the first point P21, the second point P22, the third point P23 and the fourth point P24 need not necessarily be bent and may be curved. In this case also, for example, of the curved sections, the first point P21 to the fourth point P24 may be defined as points in contact with a plane forming a 45 degree angle with respect to the extension direction.

The base end-side leg part 31 need not be directly connected to the outer tube 21, and may be connected to the outer tube 21 via a mounting member that is wound around the outer tube 21. The distal end-side leg part 35 need not necessarily be directly connected to the inner tube 22 and may be connected to the inner tube 22 via a mounting member that is wound around the inner tube 22.

### Other

Each of the balloons 3 and 7 is an example of a "balloon for a catheter" of the present invention. Each of the base end-side leg part 31 and the distal end-side leg part 35 is an example of a "joint part" of the present invention. Each of the base end-side cone part 32 and the distal end-side cone part 34 is an example of a "connecting part" of the present invention.

## Claims

1. A balloon for a catheter (3, 7) configured to inflate and deflate in accordance with changes in internal pressure, the balloon for the catheter (3, 7) comprising:
an inflation part (33, 73) extending in a cylindrical shape along an extension direction in an inflated state;
a connecting part (32, 34, 72, 74) that is a part extending from at least one end of the inflation part (33, 73), a diameter of one end of the connecting part (32, 34, 72, 74) being larger than a diameter of another end when the inflation part (33, 73) is in the inflated state, the one end being an end connected to the inflation part (33, 73) and the other end being an end on the opposite side to the one end; and
a joint part (31, 35, 71, 75) that extends from the other end of the connecting part (32, 34, 72, 74);
wherein
the connecting part (32, 34, 72, 74) includes
a first connecting part (321, 341, 721, 741) connected to the inflation part (33, 73) at a first point (P21, P41),
a second connecting part (322, 342, 722, 742) connected to the joint part (31, 35, 71, 75) at a second point (P22, P42), and
a third connecting part (323, 343, 723, 743) disposed between the first connecting part (321, 341, 721, 741) and the second connecting part (322, 342, 722, 742), connected to the first connecting part (321, 341, 721, 741) at a third point (P23, P43) and connected to the second connecting part (322, 342, 722, 742) at a fourth point (P24, P44), and
when the inflation part (33, 73) is in the inflated state, both a first angle (θ21, θ41) and a second angle (θ22, θ42) are larger than a third angle (θ23, θ43), the first angle (θ21, θ41) being an acute angle between a first line segment (L21, L41) and the extension direction, the first line segment (L21, L41) being a line segment connecting the first point (P21, P41) and the third point (P23, P43), the second angle (θ22, θ42) being an acute angle between a second line segment (L22, L42) and the extension direction, the second line segment (L22, L42) being a line segment connecting the second point (P22, P42) and the fourth point (P24, P44), the third angle (θ23, θ43) being an acute angle between a third line segment (L23, L43) and the extension direction, the third line segment (L23, L43) being a line segment connecting the third point (P23, P43) and the fourth point (P24, P44), **characterized in that**
a length (M23, M43) of the third connecting part (323, 343, 723, 743) is longer than a length (M21, M41) of the first connecting part (321, 341, 721, 741) and a length (M22, M42) of the second connecting part (322, 342, 722, 742).

2. The balloon for the catheter (3, 7) according to claim 1, wherein
the lengths (M21, M41, M22, M42) of the first connecting part (321, 341, 721, 741) and the second connecting part (322, 342, 722, 742) are substantially the same.

3. The balloon for the catheter (3, 7) according to claim 1 or 2, wherein
when the inflation part (33) is in the inflated state, a diameter of the third connecting part (323, 343) increases toward the first connecting part (321, 341).

4. A balloon catheter (10) comprising:
the balloon for the catheter (3, 7) according to any one of claims 1 to 3, and
a catheter shaft (2) extending between a base end and a distal end, the joint part (31, 35, 71, 75) of the balloon for the catheter (3, 7) being connected to the distal end.

5. A molding die (6) configured to form a balloon for a catheter (3) using blow molding, the molding die (6) comprising:
an inner wall (5) forming a cylindrical space extending in an extension direction,
wherein
the inner wall (5) includes
an inflation part (53) having substantially the same diameter along the extension direction,
a connecting part (52, 54) that is a part extending from at least one end of the inflation part (53), a diameter of one end of the connecting part (52, 54) being larger than a diameter of another end, the one end being an end connected to the inflation part (53) and the other end being an end on the opposite side to the one end, and
a joint part (51, 55) that extends from the other end of the connecting part (52, 54),
the connecting part (52, 54) includes
a first connecting part (521, 541) connected to the inflation part (53) at a first point (S21, S41),
a second connecting part (522, 542) connected to the joint part (51, 55) at a second point (S22, S42), and
a third connecting part (523, 543) disposed between the first connecting part (521, 541) and the second connecting part (522, 542), connected to the first connecting part (521, 541) at a third point (S23, S43) and connected to the second connecting part (522, 542) at a fourth point (S24, S44), and
both a first angle (U21, U41) and a second angle (U22, U42) are larger than a third angle (U23, U43), the first angle (U21, U41) being an acute angle between a first line segment (T21, T41) and the extension direction, the first line segment (T21, T41) being a line segment connecting the first point (S21, S41) and the third point (S23, S43), the second angle (U22, U42) being an acute angle between a second line segment (T22, T42) and the extension direction, the second line segment (T22, T42) being a line segment connecting the second point (S22, S42) and the fourth point (S24, S44), the third angle (U23, U43) being an acute angle between a third line segment (T23, T43) and the extension direction, the third line segment (T23, T43) being a line segment connecting the third point (S23, S43) and the fourth point (S24, S44), **characterized in that**
a length of the third connecting part (523, 543) is longer than a length of the first connecting part (521, 541) and a length of the second connecting part (522, 542).

## Patentansprüche

1. Ballon für einen Katheter (3, 7), der dafür ausgelegt ist, sich gemäß Änderungen des Innendrucks aufzublähen und zu entleeren, wobei der Ballon für einen Katheter (3, 7) Folgendes aufweist:
ein Aufblasteil (33, 73), das sich in einem aufgeblasenem Zustand in einer zylindrischen Form entlang einer Erstreckungsrichtung erstreckt;
ein Verbindungsteil (32, 34, 72, 74), bei dem es sich um ein Teil handelt, das sich ausgehend von mindestens einem Ende des Aufblasteils (33, 73) erstreckt, wobei ein Durchmesser eines Endes des Verbindungsteils (32, 34, 72, 74) größer ist als ein Durchmesser eines anderen Endes, wenn sich das Aufblasteil (33, 73) im aufgeblasenen Zustand befindet, wobei das eine Ende ein Ende darstellt, das mit dem Aufblasteil (33, 73) verbunden ist, und das andere Ende ein Ende auf der zu dem einen Ende entgegengesetzten Seite darstellt; und
ein Verbindungsstück (31, 35, 71, 75), das sich vom anderen Ende des Verbindungsteils (32, 34, 72, 74) aus erstreckt;
wobei
das Verbindungsteil (32, 34, 72, 74) umfasst:
ein erstes Verbindungsteil (321, 341, 721, 741), das mit dem Aufblasteil (33, 73) an einem ersten Punkt (P21, P41) verbunden ist,
ein zweites Verbindungsteil (322, 342, 722, 742), das mit dem Verbindungsstück (31, 35, 71, 75) an einem zweiten Punkt (P22, P42) verbunden ist, und
ein drittes Verbindungsteil (323, 343, 723, 743), das zwischen dem ersten Verbindungsteil (321, 341, 721, 741) und zweiten Verbindungsteil (322, 342, 722, 742) angeordnet ist, mit dem ersten Verbindungsteil (321, 341, 721, 741) an einem dritten Punkt (P23, P43) verbunden ist und mit dem zweiten Verbindungsteil (322, 342, 722, 742) an einem vierten Punkt (P24, P44) verbunden ist, und
wenn sich das Aufblasteil (33, 73) im aufgeblasenen Zustand befindet, sowohl ein erster Winkel (θ21, θ41) als auch ein zweiter Winkel (θ22, θ42) größer ist als ein dritter Winkel (θ23, θ43), wobei der erste Winkel (θ21, θ41) ein spitzer Winkel zwischen einem ersten Liniensegment (L21, L41) und der Erstreckungsrichtung ist, das erste Liniensegment (L21, L41) ein Liniensegment ist, das den ersten Punkt (P21, P41) und den dritten Punkt (P23, P43) verbindet, der zweite Winkel (θ22, θ42) ein spitzer Winkel zwischen einem zweiten Liniensegment (L22, L42) und der Erstreckungsrichtung ist, das zweite Liniensegment (L22, L42) ein Liniensegment ist, das den zweiten Punkt (P22, P42) und den vierten Punkt (P24, P44) verbindet, der dritte Winkel (θ23, θ43) ein spitzer Winkel zwischen einem dritten Liniensegment (L23, L43) und der Erstreckungsrichtung ist, wobei das dritte Liniensegment (L23, L43) ein Liniensegment ist, das den dritten Punkt (P23, P43) und den vierten Punkt (P24, P44) verbindet, **dadurch gekennzeichnet, dass**
eine Länge (M23, M43) des dritten Verbindungsteils (323, 343, 723, 743) länger als eine Länge (M21, M41) des ersten Verbindungsteils (321, 341, 721, 741) und eine Länge (M22, M42) des zweiten Verbindungsteils (322, 342, 722, 742) ist.

2. Ballon für einen Katheter (3, 7) nach Anspruch 1, wobei
die Längen (M21, M41, M22, M42) des ersten Verbindungsteils (321, 341, 721, 741) und zweiten Verbindungsteil (322, 342, 722, 742) im Wesentlichen identisch sind.

3. Ballon für einen Katheter (3, 7) nach Anspruch 1 oder 2, wobei,
wenn sich das Aufblasteil (33) im aufgeblasenen Zustand befindet, ein Durchmesser des dritten Verbindungsteils (323, 343) in Richtung zum ersten Verbindungsteil (321, 341) größer wird.

4. Ballonkatheter (10), aufweisend:
den Ballon für einen Katheter (3, 7) nach einem der Ansprüche 1 bis 3, und
einen Katheterschaft (2), der sich zwischen einem Basisende und einem distalen Ende erstreckt, wobei das Verbindungsstück (31, 35, 71, 75) des Ballons für einen Katheter (3, 7) mit dem distalen Ende verbunden ist.

5. Formwerkzeug (6), das dafür ausgelegt ist, einen Ballon für einen Katheter (3) mittels Blasformen zu bilden, wobei das Formwerkzeug (6) Folgendes aufweist:
eine Innenwand (5), die einen zylindrischen Raum bildet, der sich in einer Erstreckungsrichtung erstreckt,
wobei
die Innenwand (5) umfasst:
ein Aufblasteil (53), das im Wesentlichen denselben Durchmesser entlang der Erstreckungsrichtung hat,
ein Verbindungsteil (52, 54), bei dem es sich um ein Teil handelt, das sich ausgehend von mindestens einem Ende des Aufblasteils (53) erstreckt, wobei ein Durchmesser eines Endes des Verbindungsteils (52, 54) größer ist als ein Durchmesser eines anderen Endes, wobei das eine Ende ein Ende darstellt, das mit dem Aufblasteil (53) verbunden ist, und das andere Ende ein Ende auf der zu dem einen Ende entgegengesetzten Seite darstellt, und
ein Verbindungsstück (51, 55), das sich vom anderen Ende des Verbindungsteils (52, 54) aus erstreckt;
wobei das Verbindungsteil (52, 54) umfasst:
ein erstes Verbindungsteil (521, 541), das mit dem Aufblasteil (53) an einem ersten Punkt (S21, S41) verbunden ist,
ein zweites Verbindungsteil (522, 542), das mit dem Verbindungsstück (51, 55) an einem zweiten Punkt (S22, S42) verbunden ist, und
ein drittes Verbindungsteil (523, 543), das zwischen dem ersten Verbindungsteil (521, 541) und zweiten Verbindungsteil (522, 542) angeordnet ist, mit dem ersten Verbindungsteil (521, 541) an einem dritten Punkt (S23, S43) verbunden ist und mit dem zweiten Verbindungsteil (522, 542) an einem vierten Punkt (S24, S44) verbunden ist, und
sowohl ein erster Winkel (U21, U41) als auch ein zweiter Winkel (U22, U42) größer ist als ein dritter Winkel (U23, U43), wobei der erste Winkel (U21, U41) ein spitzer Winkel zwischen einem ersten Liniensegment (T21, T41) und der Erstreckungsrichtung ist, das erste Liniensegment (T21, T41) ein Liniensegment ist, das den ersten Punkt (S21, S41) und den dritten Punkt (S23, S43) verbindet, der zweite Winkel (U22, U42) ein spitzer Winkel zwischen einem zweiten Liniensegment (T22, T42) und der Erstreckungsrichtung ist, das zweite Liniensegment (T22, T42) ein Liniensegment ist, das den zweiten Punkt (S22, S42) und den vierten Punkt (S24, S44) verbindet, der dritte Winkel (U23, U43) ein spitzer Winkel zwischen einem dritten Liniensegment (T23, T43) und der Erstreckungsrichtung ist, wobei das dritte Liniensegment (T23, T43) ein Liniensegment ist, das den dritten Punkt (S23, S43) und den vierten Punkt (S24, S44) verbindet, **dadurch gekennzeichnet, dass**
eine Länge des dritten Verbindungsteils (523, 543) länger als eine Länge des ersten Verbindungsteils (521, 541) und eine Länge des zweiten Verbindungsteils (522, 542) ist.

## Revendications

1. Ballonnet pour cathéter (3, 7) configuré pour se gonfler et se dégonfler en fonction de changements de pression interne, le ballonnet pour le cathéter (3, 7) comprenant :
une partie de gonflage (33, 73) s'étendant en une forme cylindrique le long d'une direction d'extension dans un état gonflé ;
une partie de connexion (32, 34, 72, 74) qui est une partie s'étendant depuis au moins une extrémité de la partie de gonflage (33, 73), un diamètre d'une extrémité de la partie de connexion (32, 34, 72, 74) étant supérieur à un diamètre d'une autre extrémité lorsque la partie de gonflage (33, 73) est dans l'état gonflé, l'une extrémité étant une extrémité connectée à la partie de gonflage (33, 73) et l'autre extrémité étant une extrémité du côté opposé à l'une extrémité ; et
une partie raccord (31, 35, 71, 75) qui s'étend depuis l'autre extrémité de la partie de connexion (32, 34, 72, 74) ;
sachant que
la partie de connexion (32, 34, 72, 74) inclut
une première partie de connexion (321, 341, 721, 741) connectée à la partie de gonflage (33, 73) à un premier point (P21, P41),
une deuxième partie de connexion (322, 342, 722, 742) connectée à la partie raccord (31, 35, 71, 75) à un deuxième point (P22, P42), et
une troisième partie de connexion (323, 343, 723, 743) disposée entre la première partie de connexion (321, 341, 721, 741) et la deuxième partie de connexion (322, 342, 722, 742), connectée à la première partie de connexion (321, 341, 721, 741) à un troisième point (P23, P43) et connectée à la deuxième partie de connexion (322, 342, 722, 742) à un quatrième point (P24, P44), et
lorsque la partie de gonflage (33, 73) est dans l'état gonflé, à la fois un premier angle (θ21, θ41) et un deuxième angle (θ22, θ42) sont plus grands qu'un troisième angle (θ23, θ43), le premier angle (θ21, θ41) étant un angle aigu entre un premier segment de ligne (L21, L41) et la direction d'extension, le premier segment de ligne (L21, L41) étant un segment de ligne connectant le premier point (P21, P41) et le troisième point (P23, P43), le deuxième angle (θ22, θ42) étant un angle aigu entre un deuxième segment de ligne (L22, L42) et la direction d'extension, le deuxième segment de ligne (L22, L42) étant un segment de ligne connectant le deuxième point (P22, P42) et le quatrième point (P24, P44), le troisième angle (θ23, θ43) étant un angle aigu entre un troisième segment de ligne (L23, L43) et la direction d'extension, le troisième segment de ligne (L23, L43) étant un segment de ligne connectant le troisième point (P23, P43) et le quatrième point (P24, P44), **caractérisé en ce que**
une longueur (M23, M43) de la troisième partie de connexion (323, 343, 723, 743) est plus longue qu'une longueur (M21, M41) de la première partie de connexion (321, 341, 721, 741) et qu'une longueur (M22, M42) de la deuxième partie de connexion (322, 342, 722, 742).

2. Le ballonnet pour le cathéter (3, 7) selon la revendication 1, sachant que
les longueurs (M21, M41, M22, M42) de la première partie de connexion (321, 341, 721, 741) et de la deuxième partie de connexion (322, 342, 722, 742) sont sensiblement les mêmes.

3. Le ballonnet pour le cathéter (3, 7) selon la revendication 1 ou 2, sachant que
lorsque la partie de gonflage (33) est dans l'état gonflé, un diamètre de la troisième partie de connexion (323, 343) augmente vers la première partie de connexion (321, 341) .

4. Cathéter à ballonnet (10) comprenant :
le ballonnet pour le cathéter (3, 7) selon l'une quelconque des revendications 1 à 3, et
un fût de cathéter (2) s'étendant entre une extrémité de base et une extrémité distale, la partie raccord (31, 35, 71, 75) du ballonnet pour le cathéter (3, 7) étant connectée à l'extrémité distale.

5. Matrice de moulage (6) configurée pour former un ballonnet pour cathéter (3) par moulage par soufflage, la matrice de moulage (6) comprenant :
une paroi intérieure (5) formant un espace cylindrique s'étendant dans une direction d'extension,
sachant que
la paroi intérieure (5) inclut
une partie de gonflage (53) ayant sensiblement le même diamètre le long de la direction d'extension,
une partie de connexion (52, 54) qui est une partie s'étendant depuis au moins une extrémité de la partie de gonflage (53), un diamètre d'une extrémité de la partie de connexion (52, 54) étant supérieur à un diamètre d'une autre extrémité, l'une extrémité étant une extrémité connectée à la partie de gonflage (53) et l'autre extrémité étant une extrémité du côté opposé à l'une extrémité, et
une partie raccord (51, 55) qui s'étend depuis l'autre extrémité de la partie de connexion (52, 54),
la partie de connexion (52, 54) inclut
une première partie de connexion (521, 541) connectée à la partie de gonflage (53) à un premier point (S21, S41),
une deuxième partie de connexion (522, 542) connectée à la partie raccord (51, 55) à un deuxième point (S22, S42), et
une troisième partie de connexion (523, 543) disposée entre la première partie de connexion (521, 541) et la deuxième partie de connexion (522, 542), connectée à la première partie de connexion (521, 541) à un troisième point (S23, S43) et connectée à la deuxième partie de connexion (522, 542) à un quatrième point (S24, S44), et
à la fois un premier angle (U21, U41) et un deuxième angle (U22, U42) sont plus grands qu'un troisième angle (U23, U43), le premier angle (U21, U41) étant un angle aigu entre un premier segment de ligne (T21, T41) et la direction d'extension, le premier segment de ligne (T21, T41) étant un segment de ligne connectant le premier point (S21, S41) et le troisième point (S23, S43), le deuxième angle (U22, U42) étant un angle aigu entre un deuxième segment de ligne (T22, T42) et la direction d'extension, le deuxième segment de ligne (T22, T42) étant un segment de ligne connectant le deuxième point (S22, S42) et le quatrième point (S24, S44), le troisième angle (U23, U43) étant un angle aigu entre un troisième segment de ligne (T23, T43) et la direction d'extension, le troisième segment de ligne (T23, T43) étant un segment de ligne connectant le troisième point (S23, S43) et le quatrième point (S24, S44), **caractérisé en ce que**
une longueur de la troisième partie de connexion (523, 543) est plus longue qu'une longueur de la première partie de connexion (521, 541) et qu'une longueur de la deuxième partie de connexion (522, 542).
